# EUROPÄISCHE PATENTANMELDUNG

(11) **EP 4 000 618 A1**
(43) Veröffentlichungstag der Anmeldung: **25.05.2022**
(21) Anmeldenummer: 20207354.0
(22) Anmeldetag: 13.11.2020
(51) Int. Cl.: A61K 31/4196, A61K 31/635, A61K 31/15, A61K 31/16, A61K 31/21, A61K 31/352, A61K 31/375, A61K 31/44, A61K 31/517, A61K 31/60, A61K 33/26, A61K 39/395, A61K 45/06, A61P 35/00

(54) **KOMBINATION WENIGSTENS EINES HEMMSTOFFES DES XC-TRANSPORTERS MIT WENIGSTENS EINEM HEMMSTOFF DER MEMBRANSTÄNDIGEN KATALASE ZUR HEMMUNG UND/ODER INAKTIVIERUNG VON TUMORZELLEN**

(71) Anmelder: Motz, Manfred, 80689 München (DE)
(72) Erfinder: MOTZ, Manfred, 80689 München (DE); BAUER, Georg, 79104 Freiburg (DE)
(74) Vertreter: Lederer & Keller Patentanwälte Partnerschaft mbB

(57) **Zusammenfassung**

Die vorliegende Erfindung betriff eine Wirkstoff-Kombination aus wenigstens einem Hemmstoff des xC-Transporters mit wenigstens einem Hemmstoff der membranständigen Katalase zur Verwendung bei der Hemmung und/oder Inaktivierung von Tumorzellen.

## Beschreibung

Tumorzellen sind durch membranständige Katalase vor interzellulärem ROS/RNSabhängigem Apoptose-auslösenden Signaling geschützt. Die Hemmung der Katalase führt zu selektiver Tumorzellapoptose, vermittelt durch den NO/Peroxynitrit- und/oder HOCI-Weg und nachfolgende Aktivierung des mitochondrialen Wegs der Apoptose. Dabei wird Caspase-9 aktiviert, die wiederum zu nachfolgender Aktivierung von Caspase-3 führt. Nach Hemmung der Katalase muss ein kurzzeitiger Influx von H₂O₂ ins Zellinnere erfolgen, der den intrazellulären Antioxidanzstatus durch Konsum von Glutathion senkt. Dieser Influx wird durch Aquaporine vermittelt. Der xC-Transporter schleust Cystin in Zellen ein und ermöglicht dadurch die intrazelluläre Glutathionsynthese. xCT ist in der Regel auf Tumorzellen überexprimiert. Die Hemmung von xCT, z.B. durch Sulfasalazin, kann zur Ferroptose führen, einem Prozess, der analog zur Fentonchemie abläuft, an Lipidperoxiden ansetzt und unabhängig von Caspasen ist.

Der xC-Transporter wird auch als Cystin/Glutamat-Antiporter SLC7A11 bezeichnet. Dieses Transportsystem dient dazu, um Cystin für die Glutathionbiosynthese in das Zellinnere zu transportieren und wird in vielen menschlichen Tumortypen überexprimiert. Es scheint, dass die xC-Transporterfunktion bei Überexpression das Tumorwachstum fördert, teilweise weil die Ferroptose, eine Form von reguliertem Zelltod, der durch exzessive Lipidperoxidation bewirkt wird, unterdrückt wird. Ferroptose ist eine eisenabhängige Form des regulierten Zelltods, die induziert wird durch eine letale Akkumulation von Lipidperoxiden in der Zellmembran. Mechanistisch und morphologisch kann die Ferroptose von anderen Formen des regulierten Zelltods, wie Apoptose und Nekroptose, unterschieden werden.

Die vorliegende Erfindung beruht auf dem überraschenden Befund, dass die Vorbehandlung von Tumorzellen mit einem xCT-Hemmstoff, insbesondere Sulfasalazin und nachfolgende Hemmung der membranständigen Katalase, zu einem Synergieeffekt führen. Der Synergieeffekt beruht dabei nicht auf dem zu erwartenden Beitrag des Sulfasalazin zur Apoptoseinduktion mittels Ferroptose, vielmehr wird der Caspaseabhängige NO/Peroxynitritweg durch die Sulfasalazinvorbehandlung in seiner Wirkung deutlich verstärkt.

Die kombinierte Vorbehandlung mit einem xCT Hemmstoff und einem Katalaseinhibitor ermöglicht also eine neue und wirksamere Form der Tumortherapie.

Für die Tumorentstehung und Tumortherapie hat das ROS/RNS-System erhebliche Bedeutung. Reaktive Sauerstoff- und Stickstoffspezies (ROS/RNS) werden heute nicht mehr als primär ungerichtet agierende, schädigende Agenzien gesehen. Vielmehr rückt das Potenzial dieser Stoffe zu mannigfaltiger Interaktion und Signalwirkung in den Vordergrund wissenschaftlicher Analyse. Dieses Potenzial kann auch bei der Erarbeitung um therapeutische Anwendungen berücksichtigt werden.

ROS/RNS spielen bei der Tumorinitiation, Tumorpromotion, Tumorprogression, aber auch bei vielen Formen der Tumortherapie eine zentrale Rolle. Wahrscheinlich beruht dies auf folgenden Grundlagen:
1) der potenziell mutagenen Wirkung einiger Mitglieder der ROS/RNS-Familie,
2) dem Potenzial von ROS, die Proliferation von Zellen anzutreiben,
3) der Rolle von ROS bei der Aufrechterhaltung des malignen Zustands, sowie
4) der Induktion von Zelltod durch Apoptose, Nekrose oder Ferroptose.

Die Rolle der ROS/RNS und der sie kontrollierenden Enzyme wie Katalase, Superoxiddismutase, Peroxidase, NADPH-Oxidase und vielen anderen scheint immer noch widersprüchlich und zum Teil direkt zu entgegengesetzten Wirkungen zu führen.

In der Literatur wird ein weithin akzeptiertes Konzept beschrieben, wonach maligne Zellen einen höheren ROS/RNS-Stoffwechsel aufweisen sollen als nichtmaligne Zellen. Daher sollten bestimmbare Konzentrationen von ROS/RNS oder deren Dosen in vivo zu einer Situation führen, bei der die Gesamtbalance der Tumorzelle in Richtung ROS/RNS-Überschuss und damit Zelltod geht, während bei nichtmalignen Zellen und in Normalgewebe in vivo aufgrund der niedrigeren "Basis-ROS/RNS-Konzentration" die antioxidative Kapazität der Zelle das eingebrachte ROS/RNS noch wirkungsvoll unschädlich machen kann. In den Arbeiten scheint sich herauszukristallisieren, dass die Gesamtkonzentration an zellulärer Katalase, einem der zentralen antioxidativen Enzyme, im Zuge der Tumorentstehung und Progression graduell deutlich abnimmt.

Andererseits gibt es auch Literaturmeinungen, die in eine andere Richtung zeigen. So wurde das Konzept entwickelt, dass im Zuge der Tumorprogression notwendigerweise auf einen Zustand der malignen Zellen selektiert werden muss, der sich durch eine höhere Resistenz gegen exogenes Wasserstoffperoxid auszeichnet als nichtmalignes Ursprungsgewebe, aber auch die frühen Stadien maligner Zellen aus diesem Gewebe. Es konnte gezeigt werden, dass diese Resistenz durch membranständige Katalase der Tumorzellen vermittelt wird.

Vor dem Hintergrund zum Teil konträrer Literaturmeinungen wurde erfindungsgemäß von einem Konzept ausgegangen, wonach die räumliche Anordnung der analysierten Enzyme und deren Substrate, und der sich daraus ergebenden Konsequenzen betrachtet wurden.

Es ist zutreffend, dass im Zuge der Tumorentwicklung die Konzentration an Katalase im Zellinnern absinkt, mit der Konsequenz, dass z.B. H₂O₂-abhängige Signalwege im Zellinnern wirkungsvoller ablaufen können, aber auch mit der Konsequenz von mutagener Wirkung, die für die Tumorzelle eine Chance zu genetischer Vielfalt und Anpassung mit sich bringt. Es ist weiterhin zutreffend, dass eine experimentell bedingte Erhöhung der intrazellulären ROS/RNS-Konzentration für Tumorzellen in der Regel zum Zelltod über einen der möglichen Wege führt.

Andererseits wurde erfindungsgemäß berücksichtigt, dass es im Zuge der Tumorprogression zur Expression von Katalase auf der Außenseite der Zellmembran von bona fide Tumorzellen kommt. Die Katalasemoleküle sind kovalent an die Membran gebunden und liegen in einer wirksamen Konzentration vor, die effizienten Schutz vor exogenen ROS/RNS wie H₂O₂, NO und Peroxynitrit bildet. Dies ist zum Überleben der Tumorzelle auch notwendig, da sie aufgrund der Expression von NADPH-Oxidase in der Membran und NO-Synthase im Zellinnern ständig eine Mischung hochreaktiver ROS/RNS produziert, die einerseits eine Rolle für ihre Proliferation spielen, andererseits aber eine potenzielle Gefahr für das Überleben der Zelle darstellen können.

Trotz ihrer großen effektiven Wirkung gegen exogenes ROS/RNS fällt der Anteil an Katalasemolekülen auf der Zelloberfläche bei einer Gesamtbilanzierung des Katalasegehaltes einer Tumorzelle nicht ins Gewicht (und blieb daher auf diesem Weg auch unentdeckt), da die Gesamtmenge an Katalase auf der Zelloberfläche trotz hoher lokaler Dichte (und daraus resultierender Wirksamkeit) im Vergleich mit der Masse des Zytoplasmas relativ unbedeutend ist.

Gegenstand der vorliegenden Erfindung ist daher eine Wirkstoff-Kombination aus wenigstens einem Hemmstoff des xC-Transporters mit wenigstens einem Hemmstoff der membranständigen Katalase zur Verwendung bei der Hemmung und/oder Inaktivierung von Tumorzellen.

Der Hemmstoff des xC-Transporters ist bevorzugt ausgewählt aus einer Gruppe von Verbindungen umfassend Erastin, Imidazol-Keton-Erastin, Piperazin-Erastin, Sulfasalazin und Sorafenib und xC-neutralisierenden Single Domain Antikörpern.

Der Hemmstoff der membranständigen Katalase ist bevorzugt ausgewählt aus einer Verbindung umfassend die Gruppe 3-Aminotriazol, Ascorbinsäure, Salicylsäure Cyanidin, Katalase-hemmende Fab-Fragmente bzw. Single-Domain-Antikörper (Nanobodies) bzw. SOD-hemmende Fab-Fragmente bzw. Single-Domain-Antikörper, die indirekt zur Katalasehemmung führen.

In einer Ausführungsform der vorliegenden Erfindung erfolgt die Hemmung entweder des xC-Transporters und/oder der Katalase und/oder der Superoxiddismutase (SOD) mit daran bindenden Molekülen, wie Nanobodies oder Antikörperfragmenten, die spezifisch an xC, Katalase oder SOD binden.

Für die Herstellung von derartigen Antigen-bindenden Fragmenten, bei denen es sich in bevorzugter Ausführungsform um Fab-Fragmente oder um Single Domain Antikörper bzw. von Single Domain Antikörpern abgeleitete Fab-Fragmente handelt, gibt es verschiedene, dem Fachmann wohlbekannte Herstellungsverfahren. Eine wesentliche Voraussetzung ist, dass die Strukturen, gegen die die Antigen-bindenden Fragmente erzeugt werden sollen, in möglichst nativer Form vorliegend. Hier muss für möglichst schonende Präparationen gesorgt werden. Gegen diese Strukturen können entweder monoklonale Antikörper, meist durch Maus-Hybridoma-Technik, erzeugt werden. Die murinen Antikörper müssen aber charakterisiert, sequenziert und anschließend humanisiert werden, um beim Menschen eingesetzt werden zu können. Eine andere Methode der Gewinnung ist die Phase Display-Methode, bei der in vitro die gewünschten Antigen-bindenden Fragmente erzeugt werden können.

Wenn die Hemmung des xC-Transporter und/oder der Katalase oder der Superoxiddismutase bewirkt werden soll, müssen bindende Moleküle erzeugt werden, die an die jeweiligen Zielmoleküle binden. Wichtig ist dabei, dass die Zielmoleküle (xC, Katalase oder SOD) in möglichst nativer Form vorliegen, d.h. in einer Form, wie sie in der lebenden natürlichen Zelle auftritt. Dazu müssen geeignete Präparationsmethoden ausgewählt werden. An die Zielmoleküle können dann bindende Moleküle binden, die üblicherweise dadurch erzeugt werden, dass die nativen Zielmoleküle mit Bibliotheken in Verbindung gebracht werden. Durch mehrmaliges Inkontaktbringen kann die Affinität und Spezifität der bindenden Moleküle immer weiter verbessert werden, so dass am Ende bindende Moleküle vorliegen, die mit einer hohen Affinität und einer hohen Spezifität an die jeweiligen Zielmoleküle binden.

Üblicherweise ist es nicht ausreichend, nur Antigen-bindende Fragmente zu erzeugen, die an eine der Zielstrukturen (xC-Transporter, Katalase und/oder SOD) binden, sondern die Antigen-bindenden Fragmente müssen auch eine neutralisierende Wirkung aufweisen. Dies bedeutet, dass die biologische Aktivität der Zielstrukturen inhibiert oder zumindest wesentlich gehemmt werden muss. Die Testung, ob die Antigen-bindenden Fragmente die gewünschte Aktivität aufweisen, erfolgt in geeigneten in vitro-Experimenten, bei denen üblicherweise Zellen geeigneter Zelllinien eingesetzt werden.

Ein Aspekt der vorliegenden Erfindung beruht auf der zeitlichen Reihenfolge der Verabreichung der beiden Wirkstoffe. In einer Ausführungsform kann der Hemmstoff des xC-Transporters und der Hemmstoff der membranständigen Tumorzellkatalase gleichzeitig verabreicht werden.

In einer bevorzugten Ausführungsform wird jedoch der Hemmstoff des xC-Transporters zuerst verabreicht und der Hemmstoff der membranständigen Tumorzellkatalase wird erst anschließend verabreicht, wobei zwischen der Verabreichung der einzelnen Wirkstoffe ein Zeitraum liegt, der bevorzugt wenigstens 60 min und noch bevorzugter wenigstens 120 min beträgt und sich gegebenenfalls auf einen Zeitraum von bis zu 12-14 Stunden erstrecken kann.

Die Verabreichung bei in vitro-Experimenten kann leicht experimentell gesteuert werden. Bei der Verabreichung am Patienten muss die zeitliche Abfolge in Abhängigkeit von der jeweils gewählten pharmazeutischen Applikationsform angepasst werden. Wenn die Wirkstoffe oral verabreicht werden können, ist es leicht möglich, die Wirkstoffe in zeitlicher Abfolge zu nehmen. Wenn die Wirkstoffe parenteral, also beispielsweise in Form von Spritzen und/oder Infusionen verabreicht werden, müssen die zu verabreichenden Flüssigkeiten mit den jeweiligen Wirkstoffen in zeitlicher Abfolge nacheinander verabreicht werden. Da gerade bei parenteralen Verabreichungsformen mit Spritzen und/oder Infusionen einige Zeit benötigt wird, bis die Wirkstoffe an den jeweiligen Wirkort gelangen, kann die zeitliche Differenz zwischen der Verabreichung der beiden Wirkstoffe auch größer als 60 min sein.

Die erfindungsgemäße Wirkstoff-Kombination wirkt gut bei Karzinomen, insbesondere bei Magenkarzinomen.

In einer weiteren Ausführungsform werden der erfindungsgemäßen Wirkstoff-Kombination weiterhin Stoffe zugegeben, die die intrazelluläre NO-Konzentration modulieren und so zu einer Erhöhung des NO-Spiegels führen. Bevorzugt handelt es sich hierbei um einen NO-Donor.

In besonders bevorzugter Ausführungsform ist der NO-Donor ausgewählt aus der Gruppe von Verbindungen umfassend: Sodiumnitroprussid, Spermin-NONOate, PAPA-NONOate, DETA-NONOate, Nitroglycerin, S-Nitroso-N-Acetylpenicillamin, NO-Metallverbindungen.

In einer weiteren bevorzugten Ausführungsform wird der Wirkstoff-Kombination weiterhin ein spezifischer, synthetischer Ligand zugegeben, der gezielt die Superoxid-Dismutase hemmt. Besonders bevorzugt ist der Ligand, der die Superoxid-Dismutase hemmt, ausgewählt ist aus Verbindungen der Gruppe umfassend: Neutralisierende Single-Domain-Antikörper gegen SOD.

Erfindungsgemäß wurden verschiedene Experimente mit humanen Magenkarzinomzellen (MKN-45) durchgeführt. Die experimentellen Einzelheiten der Versuche sind in der Literaturstelle Heinzelmann und Bauer, Biol. Chem. (2010) 391, S. 675-693 detailliert beschrieben. Auf die experimentelle Ausgestaltung wird hiermit ausdrücklich verwiesen.

In der Literaturstelle Heinzelmann und Bauer sind die Herkunft der jeweiligen Zellen, deren Anzuchtbedingungen, die Apoptoseinduktion und deren Messung beschrieben. Die membranständige Katalase wurde mit 3-Aminotriazol (3-AT) gehemmt. Spezifische Inhibitoren, die die Differenzierung zwischen dem reaktiven NO/Peroxynitritweg und dem HOCI-Weg erlauben, sind weiterhin in der Literaturstelle Heinzelmann und Bauer (a.a.o.) offenbart. Bei den einzelnen Auswertungen wurde die Apoptoseinduktion über die Quantifizierung des Prozentsatzes derjenigen Zellen bestimmt, die die Apoptosemerkmale Kernkondensation, Kernfragmentation und/oder Membrane-Blebbing aufwiesen. Die Quantifizierung erfolgte jeweils mit Hilfe eines Phasenkontrast-Umkehrmikroskops.

Die Erfindung wird durch die Ergebnisse der Beispiele erläutert, die in den beiliegenden Figuren gezeigt sind:

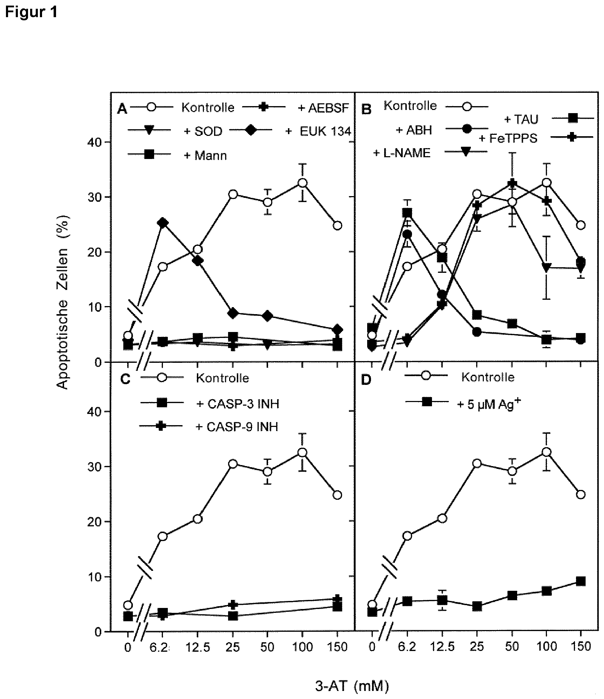

Figur 1 illustriert die Hemmung der protektiven Katalase von Tumorzellen, wodurch NO/Peroxynitrit- und HOCI-Signaling reaktiviert werden, abhängig vom mitochondrialen Weg der Apoptose und der Wirkung von Aquaporinen.
Figur 1A zeigt, dass der Katalasehemmstoff 3-Aminotriazol (3-AT) konzentrationsabhängig zur Apoptoseinduktion in humanen MKN-45 Tumorzellen führt. Die Apoptoseinduktion ist im gesamten Konzentrationsbereich von 3-AT von extrazellulären Superoxidanionen abhängig, da sie durch den Konzentrationsbereich von 3-AT von extrazellulären Superoxidanionen abhängt, da sie durch den NADPH Oxidase-Hemmstoff AEBSF und durch SOD gehemmt wird. Hydroxyradikale spielen eine essentielle Rolle, da die Apoptoseinduktion im gesamten untersuchten Konzentrationsbereich des 3-AT durch den Hydroxylradikalfänger Mannitol (Mann) gehemmt wird. H₂O₂ spielt nur bei Apoptose, die durch höhere Konzentration an 3-AT ausgelöst wird, eine Rolle, da das Katalasemimetikum EUK-134 nur in diesem Bereich zur Hemmung führt.
Figur 1B: Im niedrigen Konzentrationsbereich von 3-AT führt der NOS-Inhibitor L-NAME und der Peroxynitritfänger FeTPPS zur Hemmung, im höheren Konzentrationsbereich ist das für den Peroxidasehemmstoff Aminobenzoylhydrazide (ABH) und den HOCI-Fänger Taurin zu beobachten. In Einklang mit den bisherigen Publikationen führt eine geringfügige Hemmung der Katalase zur selektiven Reaktivierung des NO/Peroxynitrigwegs, die bei stärkerer Katalasehemmung durch den HOCI-Weg abgelöst wird.
Figur 1C: Die Apoptoseinduktion wird durch die Inhibitoren von Caspase-3 und Caspase-9 vollständig gehemmt, wodurch die Rolle des mitochondrialen Apoptosewegs belegt ist.
Figur 1D: Silberionen (5 µM) hemmen die Apoptose vollständig, was auf eine Beteiligung der Aquaporine hinweist. Aquaporine lassen nach Katalasehemmung den Influx von H₂O₂ zu und führen dadurch zu einer Abnahme des intrazellulären Antioxidanzstatus. Insgesamt zeigen diese Daten, dass nach Hemmung der membranständigen Katalase von Tumorzellen erst der NO/Peroxynitrit- und dann der HOCI-Weg reaktiviert werden. Für beide Signalwege sind Superoxidanionen und die Bildung von Hydroxylradikalen notwendig. Apoptose wird durch den mitochondrialen Weg induziert und zusätzlich durch Aquaporine moduliert.

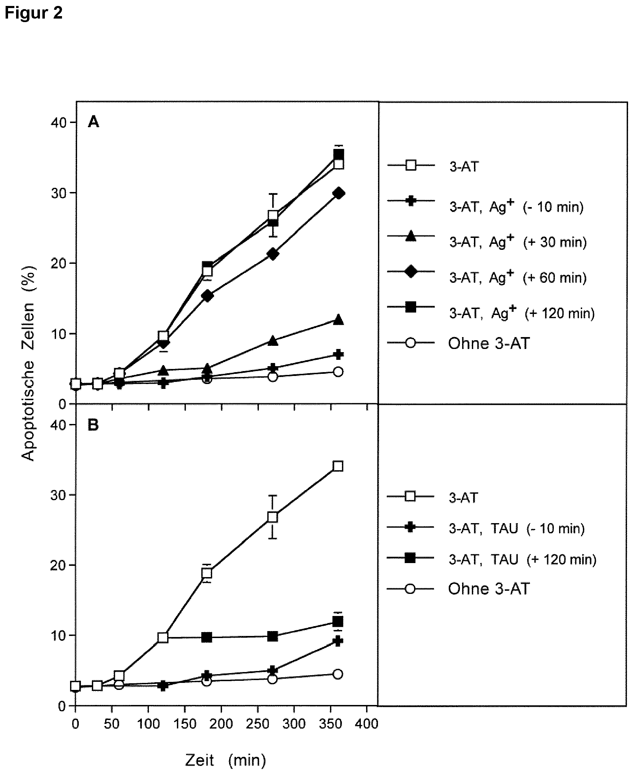

Figur 2 zeigt die Kinetik der Apoptoseinduktion in Tumorzellen nach Hemmung der Katalase.
Figur 2A: 3-AT (100 mM) führt zu einem linearen Anstieg der Apoptoseinduktion, der nach einer Lag-Phase von ca. 60 min beginnt. Zugabe von Aquaporin-hemmenden Silberionen (5 µM) vor der Gabe des Katalasehemmstoffs blockiert die Apoptoseinduktion fast vollständig. Spätere Gaben des Aquaporin-Inhibitors wirken schwächer oder gar nicht, entsprechend dem Zeitpunkt der Zugabe. Das Experiment zeigt, dass der Aquaporin-abhängige Prozess innerhalb der ersten Stunde nach Zugabe des Katalaseinhibitors abgeschlossen ist. Er ist erklärbar als Influx von extrazellulärem, aber von den Tumorzellen gebildeten H₂O₂ nach Katalasehemmung. Als Konsequenz wird der intrazelluläre Antioxidanzstatus gesenkt.
Figur 2B: Im Gegensatz zum Aquaporin-abhängigen Prozess dauert der eigentliche Signalprozess (durch den HOCI-Weg) fortwährend an, so dass auch eine Zugabe des HOCI-Fängers Taurin zwei Stunden nach Zugabe von 3-AT zu einem sofortigen Abbruch der Apoptoseinduktion führt.
   Insgesamt zeigt dieses Experiment, dass der Wirkung des reaktivierten HOCI-Wegs ein Aquaporin-abhängiger, ca. einstündiger Prozess notwendigerweise vorgelagert ist.

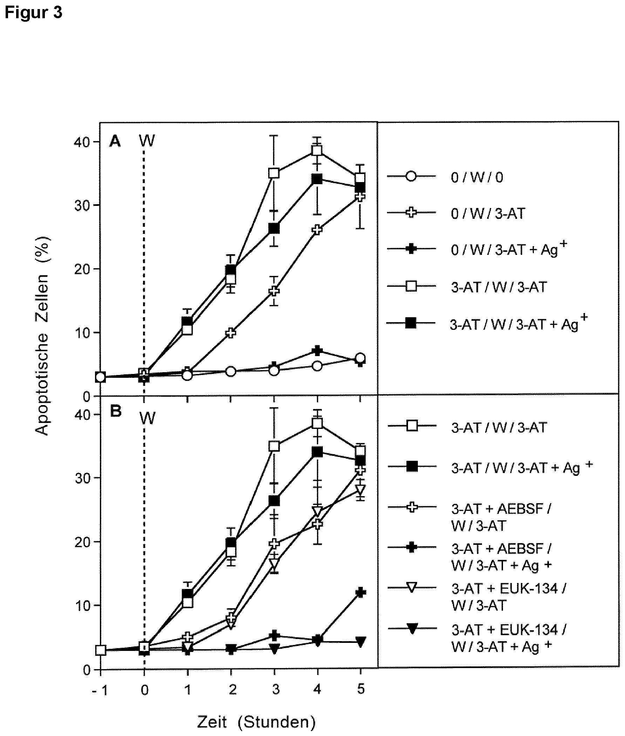

Figur 3 zeigt, dass der Aquaporin-vermittelte Schritt auf der Wirkung von Tumorzelleigenen Superoxidanionen und deren Dismutationsprodukt H₂O₂ beruht.
Figur 3A: Bei Zugabe von 100 mM 3-AT während einer einstündigen Inkubation, gefolgt von einem Waschschritt ("W") und weiterer Zugabe von 3-AT erfolgt Apoptoseinduktion ohne eine vorausgehende weitere Lag-Phase und ist außerdem von Aquaporinen unabhängig und daher nicht mehr durch Silberionen zu hemmen. Erfolgte die einstündige Vorinkubation jedoch in Abwesenheit von 3-AT, dann ist die dem Waschschritt nachfolgende Apoptoseinduktion von Aquaporinen abhängig und daher durch Silberionen zu hemmen. Zudem zeigt sich eine einstündige Lag-Phase.
Figur 3B: Wenn während der einstündigen Vorbehandlung in Gegenwart von 3-AT jedoch entweder die Produktion von Superoxidanionen mittels AEBSF gehemmt wurde oder deren Dismutationsprodukt H₂O₂ durch das Katalasemimetikum EUK-134 zerstört wurde, führt die Vorbehandlung nicht zu der unter A gezeigten Konsequenz.
   Insgesamt zeigt dieses Experiment, dass nach Katalasehemmung von Tumorzellen gebildete extrazelluläre Superoxidanionen und deren Dismutationsprodukt H₂O₂ für den Aquaporin-abhängigen Prozess verantwortlich sind. Dieser Aquaporin-abhängige Prozess hebt den Widerstand der Tumorzellen gegen die Apoptoseinduktion durch die reaktivierten Signalwege auf.
Figur 4 zeigt, dass die Vorbehandlung von Tumorzellen mit dem xCT-Hemmstoff Sulfasalazin den Aquaporin-vermittelten Effekt, der für die Apoptoseinduktion nach Katalasehemmung notwendig ist, ersetzt.
Figur 4A: Apoptoseinduktion nach Zugabe des Katalasehemmstoffs 3-AT wird durch Silberionen gehemmt, was die Abhängigkeit des Prozesses von der Aktivität von Aquaporinen belegt.

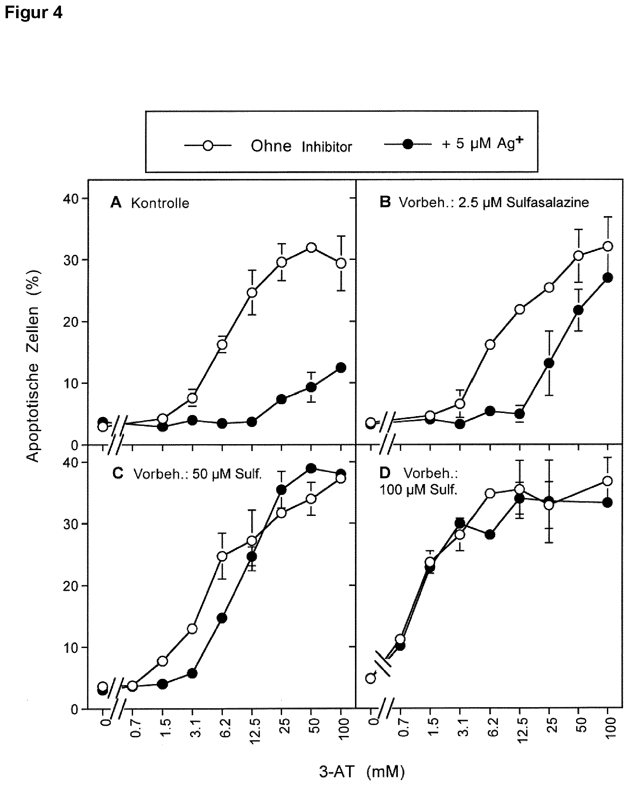

Figur 4B-D: Eine vierzehnstündige Vorbehandlung mit steigenden Konzentrationen des xCT-Hemmstoffs Sulfasalazin ersetzt graduell die Wirkung der Aquaporine, was sich in der verminderten Hemmbarkeit der Apoptoseinduktion durch Silberionen widerspiegelt. Bei einer Vorbehandlung mit 100 µM Sulfasalazin ist die Abhängigkeit von der Aquaporinwirkung vollständig aufgehoben. Außerdem macht dieses Experiment deutlich, dass die Vorbehandlung mit Sulfasalazin zu einem bisher nicht bekannten synergistischen Effekt mit der Wirkung des Katalasehemmstoffs führt. Dieser Synergieeffekt ist an der linken Flanke der gezeigten Dosiswirkungskurve sichtbar. Er erlaubt gleichbleibenden Effekt von 3-AT trotz ca. zehnfacher Absenkung deren Konzentration (nach Sulfasalazinvorbehandlung). Außerdem stellt dieses Experiment eine Kontrolle dafür dar, dass die Silberionen nicht die extrazellulären Signalwege direkt hemmen oder mit dem intrazellulären Ablauf der Apoptose interferieren.
Figur 5 zeigt, dass der Synergieeffekt zwischen Sulfasalazin und 3-AT nur erreicht wird, wenn eine Vorbehandlung der Zellen mit Sulfasalazin vorgenommen wurde.
Figur 5A: Dieses Experiment zeigt, dass die Vorbehandlung von Tumorzellen mit Sulfasalazin konzentrationsabhängig zu einem Synergieeffekt mit der Wirkung von 3-AT führt.
Figur 5B: Bei der gezeigten gleichzeitigen Gabe von Sulfasalazin und 3-AT bleibt dieser Synergieeffekt aus.
Figur 6: Die Ergebnisse, die in Figur 6 dargestellt sind, lassen erkennen, dass der Synergieeffekt zwischen Sulfasalazin-Vorbehandlung und 3-AT auf einer Verstärkung des NO/Peroxynitritsignalwegs beruht. Der differenzielle Einsatz des Peroxynitritfängers FeTPPS und des HOCI-Fängers Taurin erlaubt eine klare Diskriminierung zwischen den beiden Signalwegen.
Figur 6A: In nicht vorbehandelten Zellen wird Apoptose vor allem durch höhere Konzentrationen 3-AT ausgelöst und beruht weitgehend auf dem HOCI-Weg. Die Hemmung mittels FeTPPS zeigt einen geringfügigen Anteil des NO/Peroxynitritwegs im Bereich von 3-6 nM 3-AT. Die Steigerung der Apoptose durch Taurin im Bereich von 0.7 und 6 mM 3-AT wird durch die Aufhebung einer komplexen Konsumreaktion zwischen NO und HOCI bedingt, die zu einer relativen Erhöhung der NO-Konzentration führt.
Figur 6B: Nach Vorbehandlung mit 100 µM Sulfasalazin ergibt sich ein Synergieeffekt mit 3-AT. Die zugrundeliegende Apoptoseinduktion ist vollständig durch den NO/Peroxynitritweg bedingt, wie das Hemmprofil deutlich zeigt. Damit ist ausgeschlossen, dass der Beitrag des Sulfasalazin einen additiven Beitrag an Ferroptose bewirkt, denn Ferroptose ist unabhängig von Caspasen, während der NO/Peroxynitritweg der Aktivität von Caspase-3 und Caspase-9 bedarf.

Ohne an eine Theorie gebunden sein zu wollen, wird erfindungsgemäß von folgenden Überlegungen ausgegangen. Der xC-Transporter, ein zellulärer Transporter, der Glutamat aus den Zellen ausschleust und dafür Cystin einschleust, hat in den letzten Jahren Interesse bei der Tumorforschung hervorgerufen. Das eingeschleuste Cystin wird in den Tumorzellen mittels NADPH zu Cystein reduziert und steht dann für die Synthese von Glutathion und andere Wege zur Verfügung. Diese Wege sind offensichtlich für das

Überleben und Vermehren von Tumorzellen von besonderer Bedeutung. Daraus erklärt sich die Bedeutung der bereits beobachteten Überexpression von xC Transportern bei Tumorzellen.

Erstaunlich und bemerkenswert ist jedoch, dass dieser Zelltod nicht über klassische Apoptose- oder Nekrosewege gesteuert wird, sondern durch Ferroptose bedingt ist. Diese spezifische Form des Zelltods läuft intrazellulär ab, benötigt keine Caspasen und scheint darauf zu beruhen, dass zunächst Lipidperoxidation im Zellinnern oder an der Innenseite der Zellmembran ausgelöst wird und nachfolgend diese Lipidperoxide durch eine Reaktion mit Fe++ (analog zur Fentonreaktion) zur Bildung von Hydroxylradikalen führen. Diese richten bisher nicht im Detail charakterisierte Schäden im Zellinnern an und führen dadurch zum Zelltod. Ferroptose wird durch eine gesteigerte Aufnahme von Eisen gesteigert.

Es ist also festzuhalten, dass Apoptoseinduktion durch extrazelluläres ROS/RNS-Signaling (moduliert durch Aquaporin-vermittelte Effekte und vermittelt durch den mitochondrialen Apoptoseweg mithilfe von Caspasen) und Ferroptose nach Hemmung des xC Transporters (als strikt intrazellulärer Prozess mit Eisenbeteiligung, aber ohne Mitwirkung der Mitochondrien und Caspasen) zwei räumlich und mechanistisch vollständig voneinander verschiedene Mechanismen des Zelltods von Tumorzellen darstellen.

Andererseits, sollte der Bezug des xC Transporters zur Glutathionsynthese eine Verbindung zwischen der Hemmung dieses Transporters und der Modulation der ROS/RNS-abhängigen Apoptoseinduktion darstellen. Wenn die erfindungsgemäße Hypothese zur Wirkung des Aquaporin-vermittelten H₂O₂ -Influxes korrekt ist, sollte die Vorbehandlung der Tumorzellen mit einem xC-Transporter-Hemmstoff die Ausführung der ROS/RNS-abhängigen Apoptose von der Wirkung von Aquaporinen unabhängig machen. Es darf angenommen werden, dass durch Hemmung des xC-Transporters die Neusynthese von Glutathion verhindert wird und der Bestand an Glutathion in der Zelle aufgrund gleichzeitiger Neubildung von intrazellulärem ROS (z. B. aus fehlerhaft arbeitenden Mitochondrien) abgesenkt wird.

Figur 4 zeigt, dass die Vorbehandlung von Tumorzellen mit steigenden Konzentrationen des xCT Hemmstoffs Sulfasalazin tatsächlich dazu führt, dass die ROS/RNS-abhängige Apoptoseinduktion in steigendem Umfang von der Aktivität der Aquaporine unabhängig wird. Dies wird durch die nachlassende Inhibition durch den Aquaporin-Inhibitor Silber verdeutlicht. Dieser Befund ist zunächst nicht unerwartet, da angenommen werden darf, dass eine Absenkung des Glutathionspiegels durch Sulfasalazin ein durch Silberionen hemmbares Absenken des Glutathionspiegels durch Aquaporin-vermittelten H₂O₂ -Influx ersetzt.

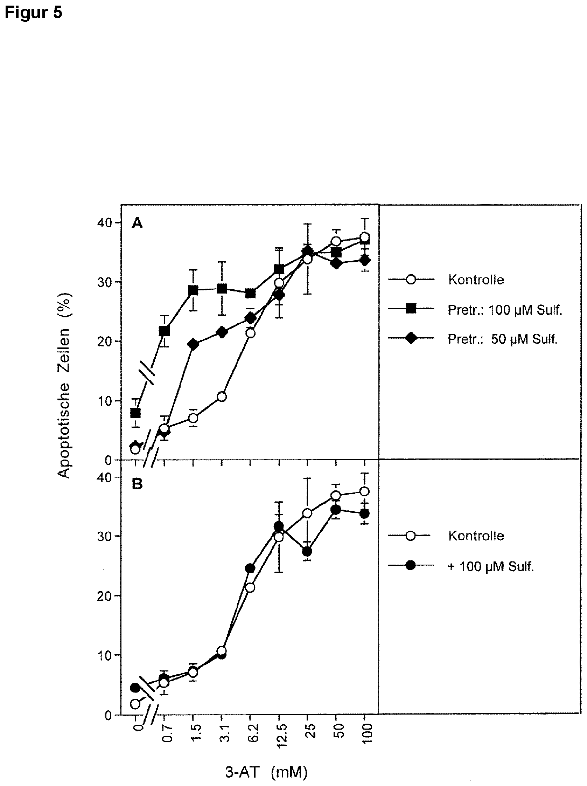

Überraschend ist auch der Befund, dass die Vorbehandlung mit Sulfasalazin nicht nur die erwartete Unabhängigkeit von der Aquaporinwirkung hervorruft, sondern auch zu einem Synergieeffekt zwischen 3-AT- und Sulfasalazinwirkung führt, wie sich aus dem Vergleich der Dosis-Wirkungsbeziehung zwischen Figur 4A und 4D sofort erkennen lässt. Figur 5 bestätigt diesen Sachverhalt in einem Wiederholungsexperiment und belegt darüber hinaus, dass die gleichzeitige Gabe von Sulfasalazin und Katalasehemmstoff nicht ausreichend für die Induktion eines Synergieeffekts ist, während durch die Vorbehandlung mit Sulfasalazin ein Synergieeffekt gemeinsam mit dem Katalasehemmstoff erreicht werden kann.

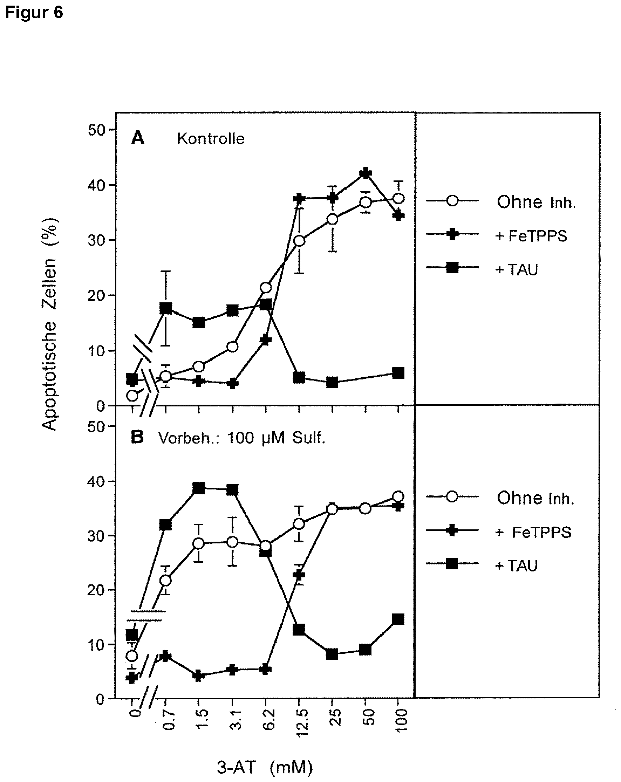

Aufgrund der unterschiedlichen Wege des Zelltods, die durch Katalasehemmung in Verbindung mit ROS/RNS-Signaling auf der einen Seite (Apoptose) und Sulfasalazinwirkung auf der anderen Seite (Ferroptose) induziert werden, könnte nun spekuliert werden, dass die Kombination von Sulfasalazinvorbehandlung mit Katalasehemmung zu einer additiven Wirkung der beidenStoffe geführt hätte. Wenn dem so wäre, so müsste der zusätzliche Anteil durch Sulfasalazin am Zelltod, der an der linken Flanke der Dosiswirkungsbeziehung zwischen 3-AT und Apoptoseinduktion erkennbar ist, vom interzellulären ROS/RNS-Signaling unabhängig sein. Die Analyse der Signalwege zeigt jedoch, dass der Synergieeffekt mit Sulfasalazin lediglich zu einer Verstärkung des NO/Peroxynitritwegs geführt hat, der auch ohne Sulfasalazin induziert wird (Figur 6). Der NO/Peroxynitritweg ist jedoch nachweislich strikt vom mitochondrialen Apoptoseweg unter Beteiligung von Caspase-9 abhängig.

Somit zeigt die Vorbehandlung mit Sulfasalazin einen nicht vorhersehbaren Synergieeffekt zwischen dem xCT-Hemmstoff und dem Katalasehemmstoff 3-AT. Dieser Synergieeffekt lässt sich nicht durch die klassische Sulfasalazinwirkung erklären, sondern ist der Verstärkung des NO/Peroxynitritwegs geschuldet.

Dieser unerwartete Effekt kann für eine verbesserte und wirkungsvollere Tumortherapie genutzt werden, bei der in Kombination mit Hemmung des xC-Transporters die Konzentrationen an Katalasehemmstoff oder Katalase-inaktivierenden Substanzen sehr deutlich gemindert werden könnte, ohne die gewünschte Wirkung zu verfehlen oder zu schmälern, Dieser Ansatz wäre in Hinblick auf das Mindern möglicher Nebenwirkungen, aber auch aus Gründen der Kostenersparnis vorteilhaft. Der starke Bezug dieses Effektes zum NO/Peroxynitritsignaling sollte zudem erlauben, die bekannten Möglichkeiten der Verstärkung dieses Weges zu nutzen, z. B. durch die gleichzeitige Gabe von exogenen NO-Donoren mit xCT-Hemmstoffen und Katalasehemmstoffen oder Stoffen, die zur Inaktivierung von Katalase führen.

### Beispiel 1

Figur 1 zeigt, wie durch Hemmung der membranständigen Katalase von Tumorzellen mittels 3-Aminotriazol (3-AT) zwei unterschiedliche Signalwege reaktiviert werden, die zur Apoptose über den mitochondrialen Apoptoseweg führen. Dies sind der NO/Peroxynitrit- und der HOCI-Weg, wobei der NO/Peroxynitritweg durch die niedrigeren 3-AT-Konzentrationen ermöglicht wird, aber bei höheren Konzentrationen an 3-AT durch den HOCI-Weg abgelöst wird.

Figur 1A belegt, dass die Apoptoseinduktion durch beide Signalwege, also im gesamten wirksamen Konzentrationsbereich von 3-AT, von Superoxidanionen abhängt, da AEBSF (*4-(2-Aminoethyl)benzenesulfonyl fluoride),* ein Hemmstoff der NADPH Oxidase zu einer vollständigen Hemmung der Apoptose führt. Ebenso führt Superoxiddismutase (SOD) (ein Enzym, das Superoxidanionen in H₂O₂ überführt) zu einer wirkungsvollen Hemmung der Apoptose. Die hemmende Wirkung von exogen zugegebener SOD belegt zudem, dass die Superoxidanionen extrazellulär agieren müssen, da extrazellulär zugegebene SOD die Membran der Tumorzellen nicht passieren kann. Die Hemmung der Apoptoseinduktion durch den Hydroxylradikal-Fänger Mannitol (MANN) zeigt die essentielle Rolle von OH-Radikalen bei diesem Prozess. Das Katalasemimetikum EUK-134 *([chloro([2,2'-[1,2-ethanediylbis[(nitrilo-κN)methylidyne]]bis[6-methoxyphenolato-κO]]]-manganese)* hemmt nur die durch höhere Konzentrationen an 3-AT induzierte Apoptose und erlaubt eine erste Differenzierung zwischen NO/Peroxynitrit- und HOCI-Weg, da nur der HOCI-Weg der Anwesenheit von H₂O₂ bedarf.

Diese Differenzierung wird in Figur 1B vertieft, da dort gezeigt wird, dass der NOS-Inhibitor L-NAME *(N-omega-nitro-L-arginine methylester hydrochloride)* und der Peroxynitrit-Scavenger FeTPPS *(5-, 10-, 15-, 20-Tetrakis(4-sulfonatophenyl)porphyrinato iron(III) chloride)* zur Hemmung der Apoptose im niedrigeren 3-AT Konzentrationsbereich führen, während der Peroxidasehemmstoff ABH (*4-Aminobenzoyl hydrazide*) und der HOCI-Fänger Taurin spezifisch Apoptose hemmen, die durch höhere Konzentrationen an 3-AT induziert wurde.

Die Daten belegen also, dass durch den Katalasehemmstoff 3-AT zwei ROS/RNSabhängige Signalwege reaktiviert werden, die im extrazellulären Raum ablaufen, sich ablösen, und zur Apoptose der Tumorzellen führen. Da in Gegenwart des Katalasehemmstoffs, aber bei Blockade der Signalwege durch entsprechende Inhibitoren, keine Apoptoseinduktion erreicht werden kann, ist gezeigt, dass intrazelluläre Katalase bei dem hier studierten Prozess keine Rolle spielt. Daher sind die beobachteten Effekte ausschließlich der Hemmung der membranständigen Katalase auf der Außenseite der Zelle zuzuschreiben. Damit in Einklang sind die Befunde, dass neutralisierende Antikörper, die nicht zellgängig sind, den gleichen Effekt auslösen wie das zellgängige 3-AT.

Figur 1C zeigt, dass Hemmstoffe von Caspase-3 und Caspase-9 zu einer vollständigen Hemmung der Apoptoseinduktion nach Katalasehemmung führen. Dies belegt die zentrale Rolle des mitochondrialen Apoptosewegs, der auf einer Aktivierung von Procaspase-9 beruht und nachfolgend zur Aktivierung von Caspase-3 führt. Aufgrund dieses Befundes ist der Zelltod als Apoptose definiert und klar von anderen Möglichkeiten des Zelltodes wie Nekrose oder Ferroptose unterschieden, da die beiden letztgenannten Wege vom mitochondrialen Apoptoseweg unabhängig sind und daher durch Caspase-9-Inhibitor nicht gehemmt werden können.

Eine wesentliche Ergänzung zum Verständnis der ROS/RNS-abhängigen Apoptoseinduktion nach Katalasehemmung zeigt Figur 1 D: Aquaporin-hemmende Silberionen (5µM) blockieren die Apoptoseinduktion vollständig. Aquaporine erlauben den Transport von Wasser durch Zellmembranen, sind aber auch von zentraler Bedeutung für den Durchgang von H₂O₂ durch diese Membranen.

### Beispiel 2

Figur 2 zeigt, dass die Hemmung der Aquaporine durch Silberionen nur dann vollständig wirkt, wenn die Silberionen vor dem Katalaseinhibitor zugegeben wurden. Die Ergebnisse der zeitlich differenzierten Zugabe der Silberionen erlauben den Schluss, dass der Aquaporin-abhängige Prozess, der modulierend auf die Apoptoseinduktion einwirkt, nur innerhalb der ersten Stunde nach Zugabe des Katalasehemmstoffs von Bedeutung ist. Das eigentliche, zum Zelltod führende interzelluläre Signaling läuft hingegen über einen größeren Zeitraum weiter. Dies ergibt sich aus der erfolgreichen und sofortigen Hemmung der Apoptoseinduktion durch Wegfangen des HOCIs mittels Taurin, auch wenn der Inhibitor erst 2 Stunden nach Beginn der Apoptoseinduktion zugefügt wird.

### Beispiel 3

Ein nachfolgendes Experiment, bei dem eine Vorbehandlung der Tumorzellen für eine Stunde, jeweils in An- oder Abwesenheit von 3-AT, mit nachfolgender Gabe von 3-AT mit oder ohne Silberionen kombiniert wurde (Figur 3A) zeigt, dass eine Vorbehandlung der Tumorzellen in Gegenwart von 3-AT zu einer Verkürzung der nachfolgenden Kinetik der Apoptoseinduktion um eine Stunde und zu einer Unabhängigkeit der Apoptoseinduktion von Aquaporinen im zweiten Teil des Experiments nach dem Waschschritt macht. Durch den Einsatz des NADPH-Oxidase-Inhibitors AEBSF und des Katalasemimetikums EUK-134 in einem analog aufgebauten Experiment (Figur 3B) kann dann gezeigt werden, dass aus Superoxidanionen generiertes H₂O₂ offensichtlich dazu führt, dass bei obligat notwendiger Katalasehemmung ausreichend H₂O₂ durch Aquaporine in die Zelle gelangt, um im späteren Verlauf der Apoptoseinduktion von der Aktivität von Aquaporinen unabhängig zu sein.

Das Ergebnisse der Beispiele 1-3, die in den Figuren 1-3 gezeigt werden, belegen, dass Tumorzellen durch membranständige Katalase vor extrazellulärem ROS/RNS-Signaling und vor dem Eindringen von H₂O₂ durch die Aquaporine geschützt werden. Folgerichtig führt die Hemmung der Katalase zu einer Reaktivierung des ROS/RNS-Signaling, Einströmen von H₂O₂ und Apoptose durch den mitochondrialen Apoptoseweg. Das Aquaporin-vermittelte Einströmen von H₂O₂ findet sehr schnell statt und ist für das Gesamtergebnis essentiell. Es ist aber für sich alleine nicht ausreichend, um Apoptose auszulösen. Dies wird dadurch belegt, dass trotz Aquaporin-vermitteltem H₂O₂-Influx Apoptose unterbunden wird, wenn das extrazelluläre ROS/RNS-Signaling gleichzeitig verhindert wurde. Außerdem ist es wichtig, im Auge zu behalten, dass trotz des Aquaporin-vermittelten H₂O₂ -Influxes die Apoptoseinduktion ausschließlich durch den mitochondrialen Weg gesteuert wird, da sie durch Caspase-9 Inhibitor vollständig hemmbar ist. Daher ist eine Rolle von Ferroptose (die Caspase-9-unabhängig durch intrazelluläres ROS ausgelöst wird) für das hier aufgezeigte System ausgeschlossen. Als Erklärung für die Rolle des H₂O₂ -Influx für das Gesamtgeschehen bietet sich im Einklang mit allen Befunden und der Literatur an, dass dadurch die Gegenwehr der Zelle gegen die Folgen der Lipidperoxidation durch extrazelluläre Hydroxylradikale vermindert wird, indem durch das einströmende H₂O₂ Glutathion konsumiert wird. Dadurch kann die Reparatur der geschädigten Membran durch das Zusammenspiel von Glutathionperoxidase-4 und Glutathion nicht mehr in ausreichendem Maß stattfinden.

## Patentansprüche

1. Wirkstoff-Kombination aus wenigstens einem Hemmstoff des xC-Transporters mit wenigstens einem Hemmstoff der membranständigen Katalase zur Verwendung bei der Hemmung und/oder Inaktivierung von Tumorzellen.

2. Wirkstoff-Kombination nach Anspruch 1, **dadurch gekennzeichnet, dass** der Hemmstoff des xC-Transporters ausgewählt ist aus einer Gruppe von Verbindungen umfassend Erastin, Imidazol-Keton-Erastin, Piperazin-Erastin, Sulfasalazin, Sorafenib, und Single Domain Antikörper, die xC neutralisieren.

3. Wirkstoff-Kombination nach einem der Ansprüche 1 oder 2, **dadurch gekennzeichnet, dass** der Hemmstoff der membranständigen Katalase ausgewählt ist aus einer Verbindung umfassend die Gruppe 3-Aminotriazol, Ascorbinsäure, Salicylsäure, Cyanidin und Moleküle, die an Katalase binden.

4. Wirkstoff-Kombination nach Anspruch 3, **dadurch gekennzeichnet, dass** die Moleküle, die an Katalase binden, ausgewählt sind aus Nanobodies und/oder neutralisierenden Single Domain-Antikörpern.

5. Wirkstoff-Kombination nach einem der Ansprüche 1 bis 4, **dadurch gekennzeichnet, dass** der Hemmstoff des xC-Transporters und der Hemmstoff der membranständigen Tumorzellkatalase gleichzeitig verabreicht werden.

6. Wirkstoff-Kombination nach einem der Ansprüche 1 bis 4, **dadurch gekennzeichnet, dass** der Hemmstoff des xC-Transporters zuerst verabreicht wird und der Hemmstoff der membranständigen Tumorzellkatalase anschließend verabreicht wird.

7. Wirkstoff-Kombination nach Anspruch 6, **dadurch gekennzeichnet, dass** der Hemmstoff des xC-Transporters zuerst verabreicht wird und dann der Hemmstoff der membranständigen Tumorzellkatalase frühestens nach 60 min verabreicht wird.

8. Wirkstoff-Kombination nach einem der Ansprüche 1 bis 7, **dadurch gekennzeichnet, dass** weiterhin Stoffe zugegeben werden, die die intrazelluläre NO-Konzentration modulieren und so zu einer Erhöhung des NO-Spiegels führen.

9. Wirkstoff-Kombination nach Anspruch 8, **dadurch gekennzeichnet, dass** zusätzlich ein NO-Donor zugegeben wird.

10. Wirkstoff-Kombination nach Anspruch 9, **dadurch gekennzeichnet, dass** der NO-Donor ausgewählt ist aus der Gruppe von Verbindungen umfassend: Sodiumnitroprussid, Spermin-NONOate, PAPA-NONOate, DETA-NONOate, Nitroglycerin, S-Nitroso-N-Acetylpenicillamin, NO-Metallverbindungen

11. Wirkstoff-Kombination nach einem der vorhergehenden Ansprüche, **dadurch gekennzeichnet, dass** weiterhin ein spezifischer, synthetischer Ligand zugegeben wird, der gezielt die Superoxid-Dismutase hemmt.

12. Wirkstoff-Kombination nach Anspruch 10, **dadurch gekennzeichnet, dass** der Ligand, der die Superoxid-Dismutase hemmt, ausgewählt ist aus Molekülen, die an SOD binden, insbesondere neutralisierenden Single Domain-Antikörpern oder Nanobodies gerichtet gegen SOD.
